# EUROPEAN PATENT SPECIFICATION

(11) **EP 4 480 527 B1**
(45) Date of publication and mention of the grant of the patent: **05.11.2025**
(21) Application number: 24210517.9
(22) Date of filing: 10.03.2022
(51) Int. Cl.: A61N 1/04, A61N 1/36, A61N 1/06

(54) **AN ELECTRODE ARRAY**
EINE ELEKTRODENANORDNUNG
UN RÉSEAU D'ÉLECTRODES

(30) Priority: 12.03.2021 US 202163160174 P; 31.03.2021 US 202163168689 P; 06.08.2021 US 202163230310 P
(43) Date of publication of application: 25.12.2024
(62) Divisional of application: 22714245.2
(73) Proprietor: Novocure GmbH, 6340 Baar (CH)
(72) Inventor: WASSERMAN, Yoram, 31905 Haifa (IL); OBUCHOVSKY, Stas, 31905 Haifa (IL); KUPLENNIK, Nataliya, 31905 Haifa (IL)
(74) Representative: Boden, Keith McMurray

(56) References cited:
- US-A- 4 722 761
- US-A1- 2013 325 096
- US-A1- 2020 171 297
- US-B1- 6 263 226

## Description

The present patent application relates to a transducer array.

### BACKGROUND

Tumor Treating Fields (TTFields or TTFs) are low intensity (e.g., 1-3 V/cm) alternating electric fields within the intermediate frequency range (100-500 kHz) targeting solid tumors by disrupting mitosis. This non-invasive treatment targets solid tumors and is described, for example, in U.S. Patent Nos. 7,016,725; 7,089,054; 7,333,852; 7,565,205; 8,244,345; 8,715,203; 8,764,675; 10,188,851; and 10,441,776.

TTFields are typically delivered through two pairs of transducer arrays that generate perpendicular fields within the treated tumor. The transducer arrays that make up each of these pairs are positioned on opposite sides of the body part that is being treated. For example, in using the OPTUNE^{®} system (manufactured by Novocure Limited, having a principle place of business in St. Helier, Jersey), at least one pair of electrodes of the transducer array is located to the left and right (LR) of the tumor, and at least one pair of electrodes is located anterior and posterior (AP) to the tumor.

Each transducer array used for the delivery of TTFields in the OPTUNE^{®} system comprises at least one set of non-conductive ceramic disk electrodes coupled to the patient's skin. For example, the OPTUNE^{®} system may position the transducer arrays on a patient's shaved head (e.g., treatment of Glioblastoma, hereinafter 'GBM') with the non-conductive ceramic disk electrodes coupled to the patient's skin through a layer of conductive medical gel.

To form the ceramic disk electrodes, a conductive layer is formed on a top surface of nonconductive ceramic material. A bottom surface of the nonconductive ceramic material is coupled to the conductive medical gel. The nonconductive ceramic material is a safety feature to ensure that direct-current signals are blocked from unintentionally being transmitted to the patient. By interposing a nonconductive ceramic material between the conductive layer and the conductive medical gel, the prior art system was thought to ensure the patient remains protected. The medical gel may deform to match the body's contours and provide electrical contact between the arrays and the skin; as such, the medical gel interface bridges the skin and reduces interference. The device is intended to be continuously worn by the patient for two to four days before removal for hygienic care and re-shaving (if necessary), followed by reapplication with a new set of arrays. As such, the medical gel remains in substantially continuous contact with an area of the patient's skin for a period of 2-4 days at a time. Further, there may only be a brief period of time in which the area of skin is uncovered and exposed to the environment before more medical gel is applied thereto.

Conventionally, the medical gel is applied manually to the electrode elements, which is a labor-intensive, tedious and expensive procedure. Further, the medical gel has a tendency to move laterally on the patient.

As such, new and improved array assemblies, and methods of making the array assemblies that speed up manufacturing and anchor the medical gel to the electrode arrays to reduce lateral movement of the medical gel is desired. It is to such assemblies and methods of producing and using the same, that the present disclosure is directed.

US-A-2020/0171297 discloses a transducer array for use in tumor-treating fields (TTFields) therapy, which has a branching configuration and includes a branching top covering, adhesive-backed layer and a skin-level adhesive layer to which electrode elements are attached.

The present invention relates to a transducer apparatus according to claim 1.

Embodiments of the invention are disclosed in the dependent claims.

### BRIEF DESCRIPTION OF THE DRAWINGS

FIG. 1 is an exemplary embodiment of a schematic diagram of electrodes applied to living tissue.
FIG. 2 is an exemplary embodiment of an electronic device configured to generate a TTField.
FIG. 3A is an exploded view of one embodiment of a transducer array that has a first layout of electrode elements.
FIG. 3B is a plan view of a flex circuit used in the transducer array illustrated in Fig. 3A.
FIG. 3C is a plan view of electrode elements used in the transducer array illustrated in Fig. 3A.
FIG. 3D is a plan view of stiffeners used in the transducer array illustrated in Fig. 3A.
FIG. 3E is a plan view of conductive hydrogel discs used in the transducer array illustrated in FIG. 3A.
FIG. 3F isa plan view of hydrogel barriers used in the transducer array illustrated in FIG. 3A.
FIG. 3G is a view of the hydrogel discs and hydrogel barriers as "seen" by the patient's skin.
FIG. 3H is a plan view of a skin-level adhesive layer used in the transducer array illustrated in FIG. 3A.
FIG. 3I is a plan view of a support layer used in the transducer array illustrated in FIG. 3A.
FIG. 3J is a plan view of the support layer of FIG. 3I connected to the top layer as "seen" by the patient's skin.
FIG. 3K is a plan view of a foam layer used in the transducer array illustrated in FIG. 3A.
FIG. 3L is a plan view of a top, covering adhesive-backed layer used in the transducer array illustrated in FIG. 3A.
FIG. 3M is a plan view of a slot cover used in the transducer array illustrated in FIG. 3A.
FIG. 3N is a plan view illustrating an appearance of a transducer array as illustrated in FIG. 3A as applied to a patient.
FIG. 3O is a plan view of a release liner used in the transducer array illustrated in FIG. 3A.
FIG. 3P is a plan view of a support layer adhered to the flex circuit with a hydrogel material.
FIG. 4 is an exploded side view of a conductive gel assembly having conductive gel layers on both sides of a support layer in accordance with the present disclosure.
FIG. 5A is a flow chart of an exemplary method of forming conductive get layers in accordance with the present disclosure.
FIG. 5B is a partial schematic view of an exemplary transducer array constructed in accordance with the present disclosure.
FIG. 5C is top plan view of use of a mold for in-line processing of multiple electrode elements.
FIG. 5D is a partial schematic view of another exemplary transducer array constructed in accordance with the present disclosure.
FIG. 6 is a flow chart of an exemplary method of using an electronic apparatus in accordance with the present disclosure.
FIG. 7A is a partial schematic view of another exemplary transducer array constructed in accordance with the present disclosure.
Fig. 7B is a side elevational view of an electrode element provided with a conductive plate and a dielectric material.
FIG. 8A is a partial schematic view of another embodiment of a transducer array constructed in accordance with the present disclosure.
FIG. 8B is a side elevational view of another embodiment of an electrode element provided with a conductive layer having a textured surface.
FIG. 9 is an exemplary embodiment of a gel application system constructed in accordance with the present disclosure.
FIG. 10 is a process for making at least one tumor treating field electrode in accordance with the present disclosure.
FIG. 11 is a process for making a transducer array in accordance with the present disclosure.

### DETAILED DESCRIPTION

Before explaining at least one embodiment of the inventive concept(s) in detail by way of exemplary language and results, it is to be understood that the inventive concept(s) is not limited in its application to the details of construction and the arrangement of the components set forth in the following description. The inventive concept(s) is capable of other embodiments or of being practiced or carried out in various ways, As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used in connection with the presently disclosed inventive concept(s) shall have the meanings that are commonly understood by those of ordinary skill in the art. Further, unless otherwise required by context, singular terms shall include pluralities and plural terms shall include the singular.

As utilized in accordance with the present disclosure, the following terms, unless otherwise indicated, shall be understood to have the following meanings:
The use of the term "a" or "an" when used in conjunction with the term "comprising" in the claims and/or the specification may mean "one," but it is also consistent with the meaning of "one or more," "at least one," and "one or more than one." As such, the terms "a," "an," and "the" include plural referents unless the context clearly indicates otherwise. Thus, for example, reference to "a compound" may refer to one or more compounds, two or more compounds, three or more compounds, four or more compounds, or greater numbers of compounds. The term "plurality" refers to "two or more."

The use of the term "at least one" will be understood to include one as well as any quantity more than one, including but not limited to, 2, 3, 4, 5, 10, 15, 20, 30, 40, 50, 100, etc. In addition, the use of the term "at least one of X, Y, and Z" will be understood to include X alone, Y alone, and Z alone, as well as any combination of X, Y, and Z. The use of ordinal number terminology (i.e., "first," "second," "third," "fourth," etc.) is solely for the purpose of differentiating between two or more items and is not meant to imply any sequence or order or importance to one item over another or any order of addition, for example.

The use of the term "or" in the claims is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used herein, any reference to "one embodiment," "an embodiment," "some embodiments," "one example," "for example," or "an example" means that a particular element, feature, structure, or characteristic described in connection with the embodiment is included in at least one embodiment. The appearance of the phrase "in some embodiments" or "one example" in various places in the specification is not necessarily all referring to the same embodiment, for example. Further, all references to one or more embodiments or examples are to be construed as non-limiting to the claims.

Throughout this application, the term "about" is used to indicate that a value includes the inherent variation of error for a composition/apparatus/ device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include"), or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB.

As used herein, the term "substantially" means that the subsequently described event or circumstance completely occurs or that the subsequently described event or circumstance occurs to a great extent or degree. For example, when associated with a particular event or circumstance, the term "substantially" means that the subsequently described event or circumstance occurs at least 80% of the time, or at least 85% of the time, or at least 90% of the time, or at least 95% of the time. For example, the term "substantially adjacent" may mean that two items are 100% adjacent to one another, or that the two items are within close proximity to one another but not 100% adjacent to one another, or that a portion of one of the two items is not 100% adjacent to the other item but is within close proximity to the other item.

The term "patient" as used herein includes human and veterinary subjects. "Mammal" for purposes of treatment refers to any animal classified as a mammal, including (but not limited to) humans, domestic and farm animals, nonhuman primates, and any other animal that has mammary tissue.

The term "liquid hydrogel" and "flowable hydrogel" as used herein may be understood to refer to an uncured hydrogel formulation that is in an at least partially flowable form. That is, the term "liquid hydrogel" refers to a hydrogel formulation prior to curing and that is curable by ultraviolet (UV) radiation or ionizing high energy radiation.

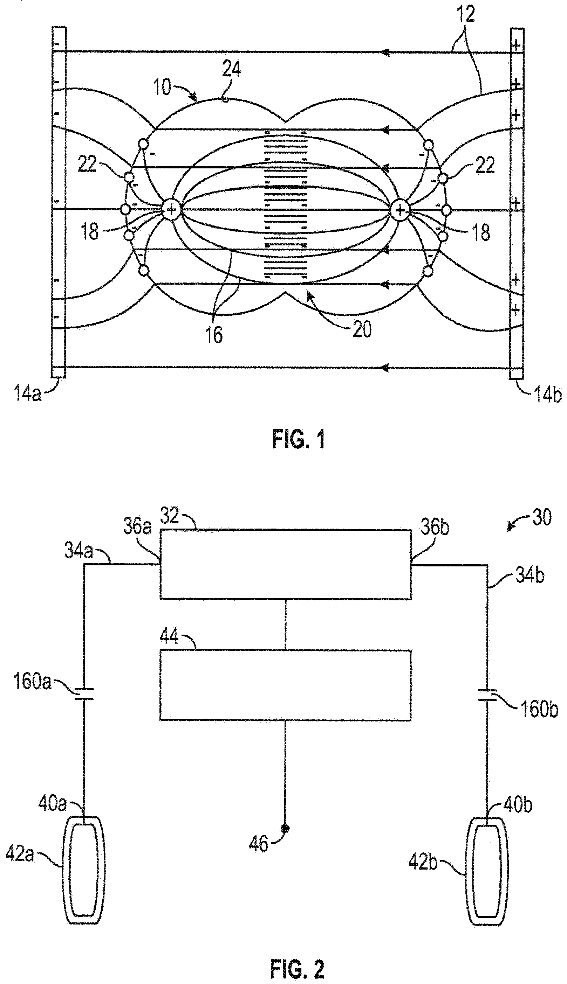

Referring now to the drawings and in particular to FIG. 1, shown therein is an exemplary embodiment of a dividing cell 10, under the influence of external TTFields 12. In some embodiments, external TTFields may include alternating fields 12 in the frequency range of about 100 KHZ to about 300 KHZ. In some embodiments, TTFields may include alternating fields 12 in the frequency range of about 50 KHZ to about 500 KHZ. In some embodiments, the TTFields may include alternating fields 12 in the frequency range of about 50KHz to about 1MHz. Fields 12 may be generated by a first electrode 14a connecting to the negative output of the electric field generator 32 and a second electrode 14b connected to the positive output. Microtubules 16, as well as other polar macromolecules within the dividing cell 10 or surrounding the dividing cell 10, may have strong dipole moment providing susceptibility to such TTFields 12. Positive outputs of the microtubules 16 may be positioned at centrioles 18. At least one negative pole may be positioned at a center 20 of the dividing cell 10 and at least one negative pole may be positioned at a point of attachment 22 of the microtubules 16 to a cell membrane 24 of the dividing cell 10. The locations of the positive outputs and the negative outputs form at least one set of double dipoles. The at least one set of double dipoles may provide susceptibility to TTFields 12 of differing directions. As used herein, the alternating electric field may be referred to as the electric field or the TTField 12. The TTField 12 may be a frequency specific, alternating electric field that is applied to the site of a tumor in the body. In some embodiments, one or more electrical fields may be applied to the dividing cell 10 in order to increase the permeability of the membrane of the dividing cell 10. For example, one or more electrical fields may be applied to the dividing cell 10 in order to increase the permeability of the membrane of the dividing cell 10 such that one or more chemicals, drugs, DNA and/or chromosomes may be introduced into the dividing cells (i.e., via electroporation).

FIG. 2 illustrates a schematic diagram of an exemplary electronic apparatus 30 configured to generate TTFields 12 in accordance with the present disclosure. The TTFields 12 described may be capable of destroying one or more tumor cells. The specifications of the electronic apparatus 30 as a whole and/or individual components of the electronic apparatus 30 as described herein may be influenced by living systems behaving according to "Ohmic' properties, rather than dielectric properties, at the frequency of the TTFields (e.g., 50 KHZ-500 KHZ or 50 KHZ -1 MHZ).

Generally, the electronic apparatus 30 may include an electric field generator 32 and two or more conductive leads 34. For example, in FIG. 2, the electronic apparatus includes a first conductive lead 34a and a second conductive lead 34b. The first conductive lead 34a includes a first end 36a and a second end 40a. The first end 36a of the first conductive lead 34a is conductively attached to the electric field generator 32 and the second end 40a of the first conductive lead 34a is connected to a first pad 42a. Similarly, the second conductive lead 34b includes a first end 36b and a second end 40b. The first end 36b of the second conductive lead 34b is conductively attached to the electric field generator 32 and the second end 40b of the second conductive lead 34b is connected to a second pad 42b. The first pad 42a and the second pad 42b may also be referred to as electrodes, such as the first electrode 14a or the second electrode 14b, or electrode pad.

The electric field generator 32 is configured to provide one or more electric signals (TTFields signals) in the shape of waveforms and/or trains of pulses as an output. Each pad 42a and 42b are provided with a potential difference by the electric signals (e.g., waveforms) that generate a current when the pads 42a and 42b are attached to a body by the electric signals (e.g., wave forms). As each of the first pad 42a and the second pad 42b is provided with the electric signals having a frequency and an amplitude, an electrical current will flow between the first pad 42a and the second pad 42b when the first pad 42a and the second pad 42b are applied on a conductive material, such as a human body.

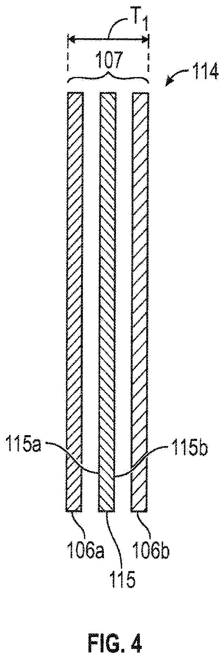

The electric field generator 32 may be configured to generate an alternating voltage wave form at frequencies in the range from about 50 KHZ to about 1 GHz, and ranges within (i.e., from about 50 KHz to about 1mHz, from about 50KHz to about 500 KHz). In some embodiments, the electric field generator 32 is configured to generate an alternating voltage wave form at frequencies in the range of about 100 KHZ to about 300 KHZ (i.e., the TTFields). The voltages are such that an electric field intensity in tissue within the treatment area is in the range of about 0.1 V/cm to about 10V/cm. To achieve this field, the potential difference between the two conductors 14, (i.e. electrode element 82 described in detail below and shown in FIG. 4) in each of the first pad 42a or second pad 42b may be determined by the relative impedances of the body.

In some embodiments, the first pad 42a and the second pad 42b may be configured to generate an alternating electric field within a target region of a patient. The target region may comprise, for example, at least a portion of a tumor. Generation of the alternating electric field may be configured to selectively destroy and/or inhibit growth of at least a portion of the tumor. The alternating electric field may be generated at any frequency capable of selectively destroying and/or inhibiting growth of at least a portion of the tumor. For example (but not by way of limitation), the alternating electric field may have a frequency within the range of about 50 kHz to about 1 mHz, as well as a range formed from any values within (i.e., a range of from about 50 kHz to about 1 mHz, a range of from about 100 kHz to about 150 kHz, a range of from about 150 kHz to about 300 kHz, etc.), and a range that combines two integers that fall between two of the above-referenced values (i.e., a range of from about 32 kHz to about 333 kHz, a range of from about 78 kHz to about 298 kHz, etc.).

In some embodiments, the alternating electric field may be configured to be imposed at two or more different frequencies. In some embodiments, each of the two or more different frequencies may be selected from any of the above-referenced values, or a range formed from any of the above-referenced values, or a range that combines two integers that fall between two of the above-referenced values.

In some embodiments, the first pad 42a and the second pad 42b (i.e., a pair of pads) may be configured differently depending upon the application in which the pair of pads 42a and 42b are to be used. In some embodiments, the pair of pads 42a and 42b may be externally applied to a patient (e.g., applied to an epidermis layer of skin of a patient) with the generation of the electric field (TTField) provided within tissue of the patient. Generally, each of the first pad 42a and the second pad 42b is placed on the epidermis of the skin of the patient by a user such that the electric field is configured to generate across tissue of a patient within a predetermined treatment area. TTFields that are applied externally can be of a local type or widely distributed type, for example, the treatment of skin tumors and treatment of lesions close to the skin surface.

In some embodiments, the user may be a medical professional, such as a doctor, nurse, therapist, or other person acting under the instruction of a doctor, nurse, or therapist. In some embodiments, the user may be the patient, that is, the patient may place the pads 42a and 42b on the epidermis layer within a predetermined treatment area.

In some embodiments, the electronic apparatus 30 may optionally include a control box 44 and one or more temperature sensor 46 coupled to the control box 44. In some embodiments, multiple temperature sensors 46 may be positioned to sense temperature at the predetermined treatment area. The one or more temperature sensor 46 may include, but are not limited to, thermistors, thermocouples, RTDs, integrated circuit temperature sensors such as the Analog Devices ADS90 and the Texas Instruments LM135, and/or combinations thereof. It is contemplated that any temperature sensor 46 known within the art may be used if configured to provide an accurate and/or precise temperature reading of the predetermined treatment area. The control box 44 may be configured to control amplitude of the electric field so as not to generate excessive heating in the treatment area.

In some embodiments, the control box 44 may be configured to control output of the electric field generator 32. For example, in some embodiments, the control box 44 may be configured to control output of the electric field generator 32 such that output remains constant at a value preset by a user. In some embodiments, the control box 44 may be configured to set output of the electric field generator 32 at a maximal value, with the maximal value configured such that excessive heat is not provided at the predetermined treatment area. In some embodiments, the control box 44 may be configured to provide one or more feedback indicators. For example, the control box 44 may be configured to provide a feedback indicator (e.g., sound, light) when a temperature of the predetermined treatment area (as sensed by temperature sensor 746) exceeds a preset limit.

In some embodiments, the control box 44 may be configured to control output of the electric field generator 32 based on one or more readings of the temperature sensor 46. In some embodiments, one or more temperature sensor 46 may be connected to and/or otherwise associated with the first pad 42a or the second pad 42b and configured to sense temperature of the epidermis and/or treatment area at either one or both of the first pad 42a or the second pad 42b.

In some embodiments, one or more of the conductive leads 34 may be standard isolated conductors having a flexible metal shield. In some embodiments, the flexible metal shield may be grounded to prevent spread of any electric field generated by the one or more conductive leads 34.

Pads 42a and 42b may be shaped, sized and positioned to generate the TTField configuration, direction and intensity at the treatment area. To that end, the pads 42a and 42b may be square, rectangular, circular, oval, or any fanciful shape.

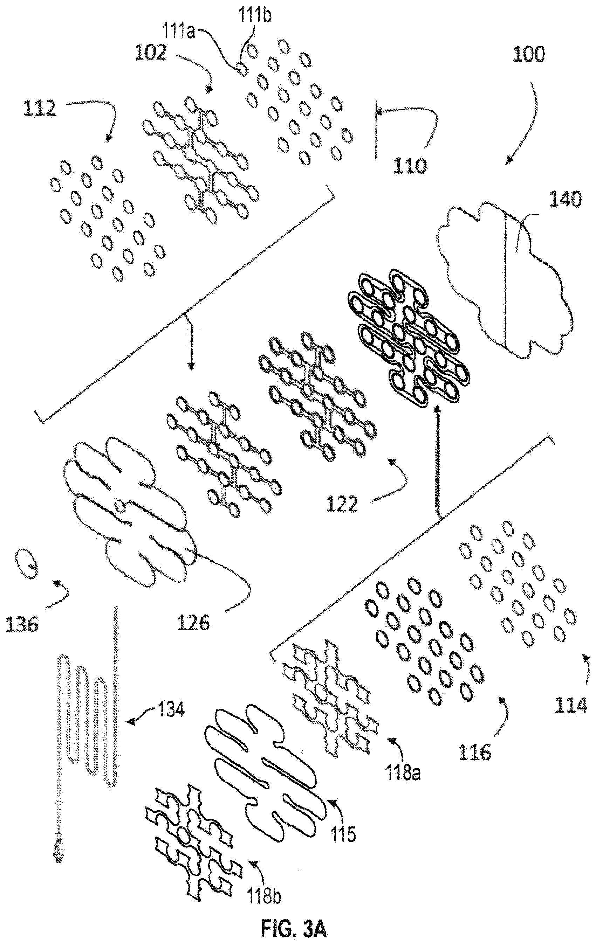

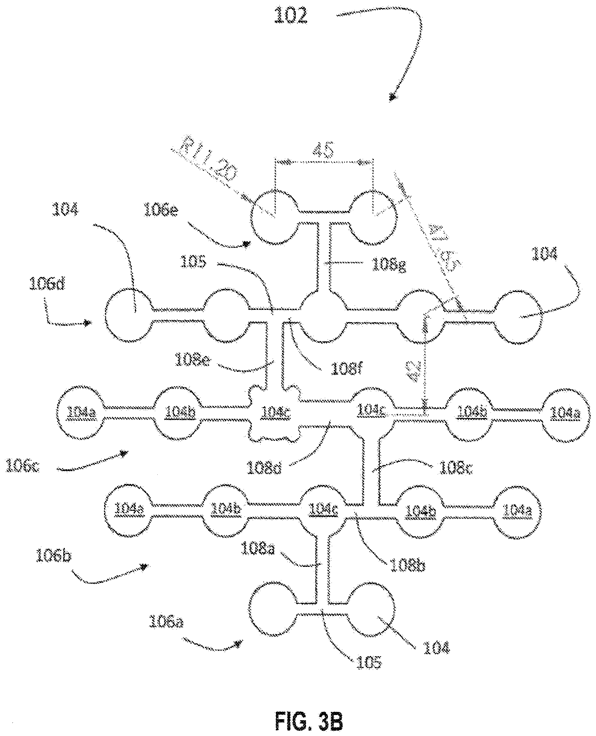

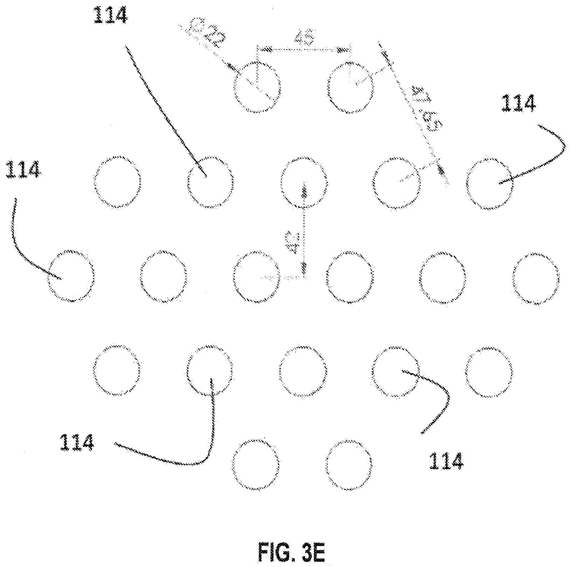

FIGS. 3A - 3P depict a first embodiment of a transducer array 100 incorporating the pads 42a and 42b. The pads 42a and 42b are referred to below as "electrode elements." The transducer array 100 is also referred to herein as an "electrode apparatus" with a first layout of electrode elements, with FIG. 3A being an exploded or assembly view showing all of the various components and their arrangement relative to each other. FIGS. 3B-3P show the individual components in greater detail.

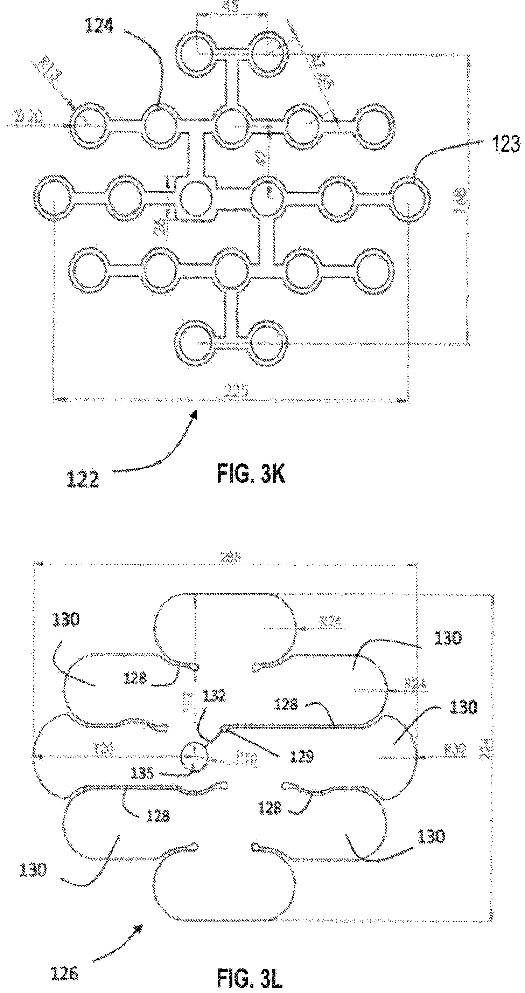

One of the components that defines the configuration of the transducer array 100 is the flex circuit 102 (FIGS. 3A and 3B), which may be made with electrical traces extending along the branches of the flex circuit 102 as is well known in the art. The flex circuit 102 has a branching or ramified configuration. There is a central trunk 108a-108g that extends in a longitudinal direction. There are also a plurality of branches that extend laterally from both sides of the trunk 108a-g of the flex circuit 102. In some embodiments (including the embodiments depicted in FIGS. 3A, 3B, and 3K), these branches are perpendicular to the longitudinal direction and are arranged as rows 106a-106e of the flex circuit 102. In the illustrated embodiment, each of the rows 106a-e of the flex circuit 102 includes two branches - one on either side of the trunks 108a-108g. The proximal end of each branch is connected to and extends from the trunk 108a-g of the flex circuit 102, while the distal end of each branch remains free. Advantageously, this configuration improves the flexibility of the flex circuit 102, and reduces tensile stresses applied on the transducer array 100 by skin movement (bending, stretching, twisting, breathing, etc.), thereby improving and prolonging adhesion of the transducer array 100 to the skin. The transducer array 100 also improves user comfort and reduces skin damage. Note that in the embodiment illustrated in FIGS. 3A and 3B, the trunk 108a-108g shifts back and forth in segments between successive rows of the flex circuit 102. In these embodiments, only some of the segments 108a, 108c, 108e, and 108g extend in the longitudinal direction, and those longitudinal segments are interconnected by additional segments 108b, 108d, and 108f that extend in the lateral direction. As a result, in these embodiments, the trunks 108b, 108d, and 108f shift back and forth in the lateral direction as the trunks 108a, 108c, 108e and 108g extend in the longitudinal direction. In alternative embodiments, the trunk is straight.

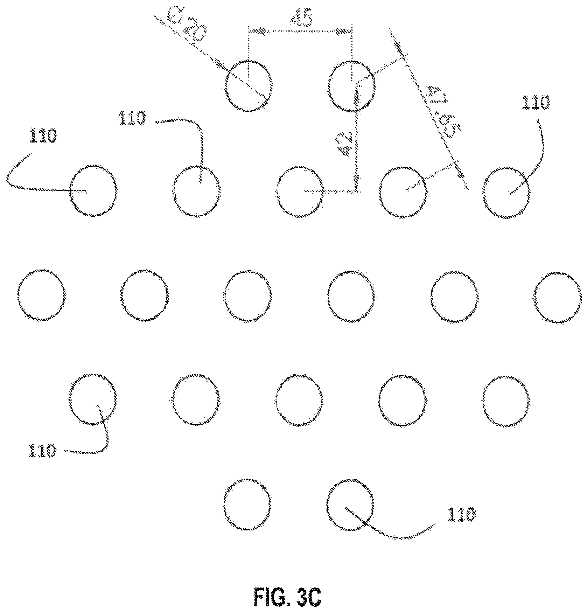

The flex circuit 102 includes a number of mounting pads 104 arranged along the rows 106a-106e. A number of electrode elements 110 (FIGS. 3A and 3C) - for example, twenty as shown in the FIG. 3A embodiment for a typically sized adult male - are disposed on the inner (i.e., skin-facing) side of the mounting pads 104 of the flex circuit 102 (shown in FIGS. 3A and 3B) with an electrically conductive connection between each of the electrode elements 110 and the flex circuit 102. The electrode elements 110 may be on the order of 1 mm thick and 2 cm in diameter and may optionally be slightly smaller in diameter than the mounting pads 104. Each of the electrode elements 110 may be formed from a circular conductive plate that is coated with a ceramic dielectric material as is known in the art, and the circular conductor is electrically connected to an electrical contact of the flex circuit 102. The ceramic dielectric material faces toward the patient's body so that the ceramic dielectric material can make contact with the patient's skin (preferably via an intervening layer of hydrogel, as described below). The dielectric material may be a ceramic material, a non-flexible polymer, or a flexible polymer film.

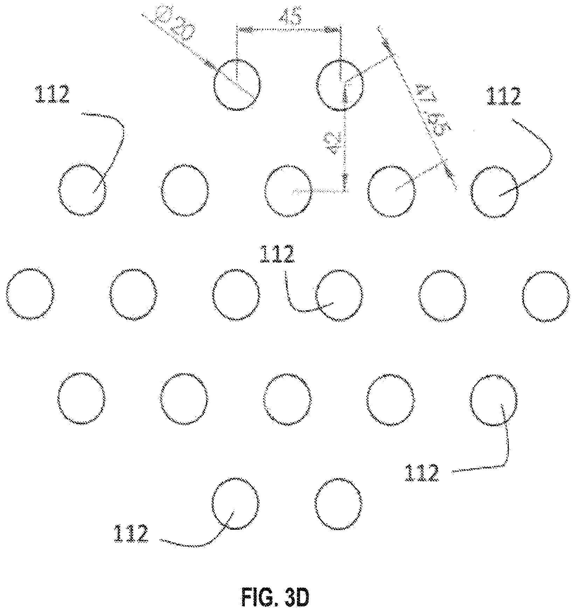

The electrode elements 110 are provided with an outer side 111a and an inner side 111b. Only one of the electrode elements 110 is labeled with the reference numerals 111a and 111b for purposes of brevity. A corresponding number of stiffeners 112 (FIGS. 3A and 3D) may optionally be attached to the outer side 111a of the mounting pads 104 of the flex circuit 102 generally opposite the electrode elements 110 attached to the inner side 111b. The stiffeners 112 may be on the order of 1 mm thick and may be slightly smaller in diameter than the mounting pads 104. The stiffeners 112 may be made from any suitable material (e.g., a stiff, nonconductive plastic). In general, the stiffeners 112 help prevent the electrode elements 110 from breaking, given the flexible nature of the flex circuit 102 and the thin, fragile nature of the ceramic dielectric used for the electrode elements 110.

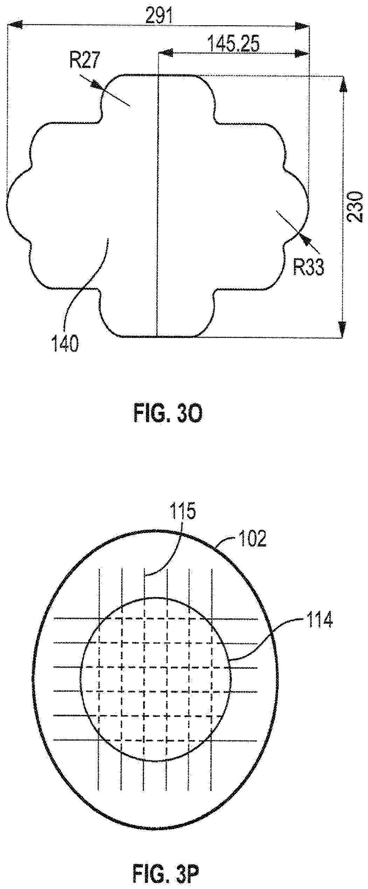

In some embodiments, each of the electrode elements 110 has a corresponding disc of conductive gel element 114 (FIGS. 3A, 3P, and 4) disposed on the inner side 111b of the electrode element 110, to establish good electrical conductivity with the patient's skin. In some embodiments, the conductive gel element 114 is slightly larger in diameter than the electrode element 110. The material is preferably gamma sterilization-compatible. For example, the conductive gel element 114 may be a hydrogel made from AG625, which is available from Axelgaard, with a thickness on the order of 635 micrometers, and with a volume resistivity of 1000 ohm-cm max. In some embodiments, the conductive gel element 114 includes one or more conductive gel layer 106 (see Fig. 4).

In some embodiments, the electrode array includes a conductive gel assembly 107, including one or more conductive gel element 114 and a support layer 115 connected to the one or more conductive gel element 114. The conductive gel element 114 includes one or more conductive gel layers 106 that may be prefabricated prior to inclusion on the electrode elements 110. In some embodiments, one or more conductive gel layers 106 may be applied in liquid form onto the electrode elements 110 and then cured (e.g., UV curing, electron beam curing) directly on the electrode element 110 and/or other portion of the transducer array 100. The support layer 115 can be applied to cover the conductive gel layer 106 and the one or more electrode elements 110, and another amount of conductive gel in liquid form or cured form may be applied to the support layer 115 so that the conductive gel layers 106 forming one of the conductive gel elements 114 are aligned and sandwich the support layer 115. In some embodiments, one or more conductive gel layers 106 may be cured directly on the support layer 115, for example and then subsequently applied to the electrode elements 110. The support layer 115 includes a first side 115a and a second side 115b. The support layer 115 is sized and dimensioned to extend over one or more electrode elements 110. The conductive gel layer 106a is disposed upon and attached to the first side 115a of the support layer 115. The conductive gel layer 106b is disposed upon and attached to the second side 115b of the support layer 115. The support layer 115 can be disposed on both of the first side 115a and the second side 115b to encapsulate a portion of the support layer 115 between the conductive gel layers 106a and 106b. The conductive gel assembly 107 can be manufactured separately from the other components of the transducer array 100 and subsequently connected to the transducer array 100. Or, the conductive gel assembly 107 can be manufactured with the transducer array 100, such as by forming the conductive gel layer(s) 106 on the electrode elements 110 either before or after application of the support layer 115 over the electrode elements 110.

The bulk electron transport agent(s) may be any substance that is capable of enhancing the electrical and/or thermal conductivity of the conductive gel. In certain non-limiting embodiments, the bulk electron transport agent(s) includes one or more ionic compounds, one or more metals, or one or more non-metals, as well as any combinations thereof. In certain non-limiting embodiments, the bulk electron transport agent comprises an amorphous carbon and/or a crystalline carbon. Particular (but non-limiting) examples of bulk electron transport agents that may be utilized in accordance with the present disclosure include carbon black, graphene, and graphite.

In some embodiments, the conductive gel element 114 and/or the one or more conductive gel layer 106 are formed primarily of a conductive gel or semi-solid conductive gel such as described below. The conductive gel element 114 may be in any form that allows the electrode elements 110 to function in accordance with the present disclosure. For example (but not by way of limitation), the conductive gel element 114 may be in the form of a hydrogel or a hydrocolloid.

The conductive gel element 114 may have properties including, but not limited to, high conductivity, tackiness, and/or biocompatible for extended periods of time. For example, the conductive gel element 84 may include AG603 Hydrogel, available from AmGel Technologies, having a principle place of business in Fallbrook, California.

The conductive gel element 114 may be used with modified hydrogels, including but not limited to perforations, recesses, and/or protrusions. Such features are further disclosed in detail in U.S. Patent Publication No. 2021/0346693.

In some embodiments, the conductive gel element 114 may be sterile. In some embodiments, the conductive gel element 114 may be configured such that the conductive gel element 114 does not substantially degrade upon exposure to sterilization conditions that include gamma rays or ethylene oxide gas, for example.

The conductive gel element 114 may be formed of any hydrophilic polymer that allows the conductive gel element 114 to function in accordance with the present disclosure. For example (but not by way of limitation), the conductive gel element 114 may be a polyacrylic acid gel, a povidone gel, or a cellulose gel. In addition, the conductive gel may comprise at least one of chitosan, alginate, agarose, methylcellulose, hyaluronan, collagen, laminin, matrigel, fibronectin, vitronectin, poly-1- lysine, proteoglycans, fibrin glue, gels made by decellularization of engineered and/or natural tissues, as well as any combinations thereof. Further, the conductive gel element 84 may comprise at least one of polyglycolic acid (PGA), polylactic acid (PLA), poly-caprolactone (PCL), polyvinyl alcohol (PVA), polyethylene glycol (PEG), methyl methacrylate, poly(methyl methacrylate) (PMMA), poly(2- hydroxyethyl methacrylate) (PolyHEMA), poly(glycerol sebacate), polyurethanes, poly(isopropylacrylamide), poly(N-isopropylacrylamide), or any combination thereof.

In some embodiments, the conductive gel element 114 may comprise one or more of the following chemical and structural features/properties: a polymer chain length in a range of from about 1 nm to about 200 nm; a pH in a range of from about 6 to about 8; a volume resistivity of less than about 100 Ohm-in; a skin adhesion rate of at least about 100 g/inch; and a thickness in a range of from about 10 mil to about 50 mil.

In some embodiments, the conductive gel element 114 may be optimized for use at body temperature (i.e., in a range of from about 34°C to about 40°C) for extended periods of time.

The polymer(s) of the conductive gel element 84 may be provided with any polymer chain length that allows the conductive gel element 114 composition(s) to function as described herein. For example (but not by way of limitation), the polymer chain length may be within the range of about 1 nm, to about 200 nm, and above, as well as a range that combines any two of the values within (i.e., a range of from about 3 nm to about 175 nm, a range of from about 5 nm to about 150 nm, or a range of from about 10 nm to about 125 nm, a range of from about 15 nm to about 100 nm, etc.), and a range that combines two integers that fall between two of the above-referenced values (i.e., a range of from about 3 nm to about 157 nm, etc.).

In some embodiments, the range of the polymer chain length may be dependent upon the frequency(ies) of the alternating electric field. For example (but not by way of limitation), the range of the polymer chain length may be based upon a range of frequencies of the alternating electric field. Non-limiting examples include a range of from about 5 nm to about 50 nm when the alternating electric field has a frequency in a range of from about 50 kHz to about 150 kHz, a range of from about 50 nm to about 100 nm when the alternating electric field has a frequency in a range of from about 150 kHz to about 300 kHz, etc.

The conductive gel element 114 may be provided with any pH that does not damage the skin of a patient. For example (but not by way of limitation), the conductive gel element 84 may have a pH of about 6, about 6.5, about 7, about 7.5, about 8, as well as a range formed from any of the above values (i.e., a range of from about 6 to about 8, a range of from about 6.5 to about 7.5, etc.).

The conductive gel element 114 may be provided with any level of volume resistivity that maximizes the conductivity of the gel. For example (but not by way of limitation), the conductive gel element 114 may have a volume resistivity within a range of less than about 100 Ohm-in to less than about 10 Ohm-in, or lower, as well as a range formed of any of the above values within (i.e., a range of from about 10 Ohm-in to about 100 Ohm-in, etc.) and a range that combines two integers that fall between two of the above-referenced values (i.e., a range of from about 13 Ohm-in to about 96 Ohm-in, etc.).

The conductive gel element 114 may be provided with any skin adhesion rate that allows the conductive gel element 114 to function in accordance with the present disclosure. For example (but not by way of limitation), the skin adhesion rate of the gel may be within a range of at least about 100 g/inch to at least about 300 g/inch, or higher, as well as a range of any of the above values within (a range of from about 120 g/inch to about 300 g/inch, etc.), and a range that combines two integers that fall between two of the above-referenced values (i.e., a range of from about 115 g/inch to about 295 g/inch, etc.).

In some embodiments, the conductive gel element 114 may further include at least one additive. Any type of additive that allows the conductive gel element 114 to function in accordance with the present disclosure and that may optionally further enhance the conductivity and non-sensitizing properties of the conductive gel may be utilized in accordance with the present disclosure. Non-limiting examples of additives that may be utilized include at least one of a humectant, a preservative, an antibacterial agent, a vitamin, a moisturizer, or any combinations thereof, and the like.

The conductive gel element 114 may be provided with any concentration of one or more salts that allow gel compositions to function as described herein. The free salt concentration may be with the range of at least about 0.1 mM to about 1M, or higher, as well as any range that combines any two of the values within (e.g., range of about 0.1 mM to about 100mM, a range of about 1mM to about 50 mM).

The conductive gel assembly 107 may be provided with any thickness t₁ that allows the conductive gel element 84 to function in accordance with the present disclosure. Non-limiting examples of thicknesses t₁ that may be utilized in accordance with the present disclosure include a range of about 1 mil to about 100 mil, or higher, as well as a range that combines any two of the above-referenced values (i.e., a range of from about 10 mil to about 50 mil, etc.), and a range that combines two integers that fall between two of the above-referenced values (i.e., a range of from about 12 mil to about 48 mil, etc.).

In some embodiments, the conductive gel assembly 107 and the conductive gel element 114 may have a shelf life of at least about six months. For example (but not by way of limitation), the conductive gel element 114 has a shelf life of at least about 9 months or at least about 12 months.

The one or more support layers 115 may be provided within the conductive gel element 114 (as shown in Fig. 4) or outside of the conductive element as shown in Fig. 3A. Generally, the one or more support layers 115 may be configured to provide reinforcement to the conductive gel element 114 to prevent lateral movement of the conductive gel element 114 when the conductive gel element 114 is applied to the skin of the patient. To that end, the one or more support layer 115 may provide strength and/or support to the one or more conductive layer 106 (e.g., conductive gel layers 106a and/or 106b). The one or more support layer 115 may be constructed of a woven or nonwoven material. In some embodiments, at least a portion of the one or more support layer 115 may include Reemay (i.e., spun nylon). In some embodiments, at least a portion or the entirety of the one or more support layer 115 may be constructed of a nonconductive material. In some embodiments, at least a portion or the entirety of the one or more support layer 115 may be constructed of a non-metal conductive material, such as carbon.

The support layer 115 may serve as an anchor between the conductive gel layer(s) 106 and one or more other components within the transducer array 100, such as the electrode elements 110 or the flex circuit 102 by attaching the support layer to the electrode elements 110 or the flex circuit 102, for example with a bond separate from any bond provided by the conductive gel layer(s) 106. In some embodiments, at least a portion of the support layer 115 may extend between at least two components of the transducer array 100, such as the electrode elements 110, or the conductive gel elements 114. For example, as illustrated in FIG. 3A, the support layer 115 extends between the electrode elements 110 and the conductive gel elements 114. In some embodiments, the conductive gel elements 114 may be positioned such that each conductive gel element 114 is aligned with one electrode element 110.

A gap may exist between each electrode element 110 such that each electrode element 110 is positioned at a distance from any adjacent electrode element 110. To that end, the conductive gel in liquid form or semi-solid conductive gel forming the conductive gel layer 106a and/or 106b may be dispensed onto the support layer 115 at pre-determined particular locations during formation of the transducer array 100 (e.g., corresponding to a single electrode element 110, corresponding to two electrode elements 110, corresponding to multiple electrode elements 110, and the like). In some embodiments, one or more dielectric materials may be positioned within the gap between adjacent isolated electrode elements 110, for example.

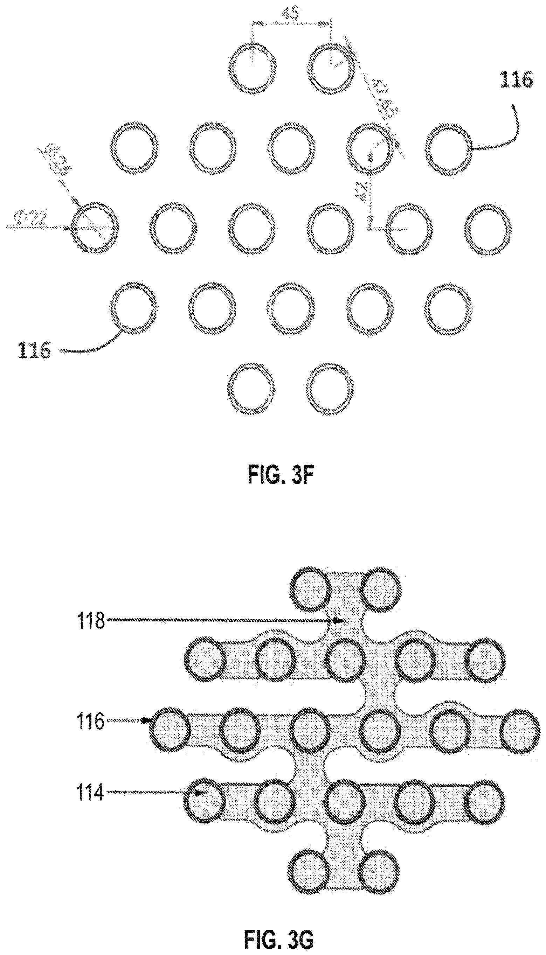

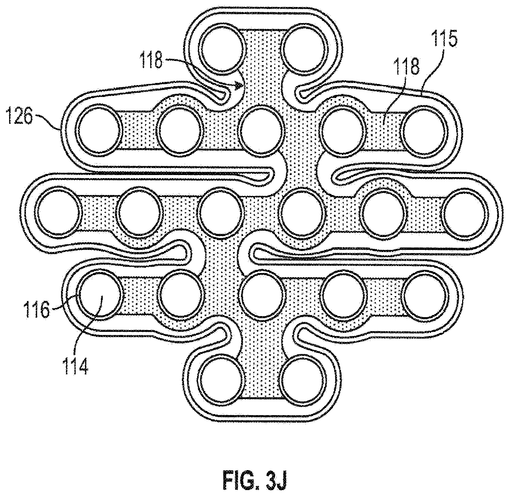

Additionally, a ring-shaped hydrogel barrier 116 (FIGS. 3A, 3F, 3G and 3J) is optionally provided, surrounding each of the conductive gel elements 114. In general, the hydrogel barriers 116 help maintain the integrity of the conductive gel elements 114 throughout the duration of wear and prevent migration of the hydrogel from its correct location under the electrode elements 110. The hydrogel barriers 116 may be made, e.g., from MED S695R, available from Vancive Medical Technologies, which is a polyethylene foam, and may be single-coated with WetStickTM synthetic rubber adhesive, also available from Vancive Medical Technologies, The hydrogel barriers 116 may be 500 micrometers thick, and are preferably gamma sterilization-compatible.

To increase patient comfort, the transducer array 100 may optionally include a conformal foam layer 122 (FIGS. 3A and 3K) positioned beneath the flex circuit 102, and shaped to closely follow the branching configuration of the flex circuit 102, Note that unlike the flex circuit 102 (which has solid circular mounting pads 104 for the electrode elements 110), the foam layer 122 has ring-shaped regions 123 that surround the electrode elements 110 so as not to intervene between the electrode elements 110 and the patient's skin. A suitable thickness for the conformal foam layer 122 is on the order of 1 mm, and the foam layer 122 is preferably the same thickness as the electrode elements 110. The foam layer 122 preferably covers the entire surface of the flexible flex circuit 102 (except for the regions where the electrode elements 110 are positioned) while maintaining overall flexibility and conformability of the transducer array 100. But in alternative embodiments, the foam layer 122 only covers a portion of the surface of the flexible flex circuit 102. In some embodiments, the size of the foam layer 122 may be minimized to the extent possible so as not to reduce the overall breathability and fluid-vaporizing properties of the transducer array 100.

The conformal foam layer 122 may be made, e.g., from polyethylene foam such as MED 5696R available from Vancive Medical Technologies. The conformal foam layer 122 may be affixed to the flex circuit 102 using a suitable adhesive (e.g., WetStickTM synthetic rubber adhesive, also available from Vancive Medical Technologies). The foam layer 122 advantageously protects the patient from potentially sharp edges of the conductive traces on the flex circuit 102. This is particularly important in the context of flexible transducer arrays because flexing the transducer arrays 100 can cause the flat conductive traces to twist, which can cause the potentially sharp edges of those conductive traces to tilt down towards the patient's skin. Notably, interposing the foam layer 122 between the conductive traces of the flex circuit 102 and the patient's skin protects the patient from cuts and/or pain that might be caused by those potentially sharp edges.

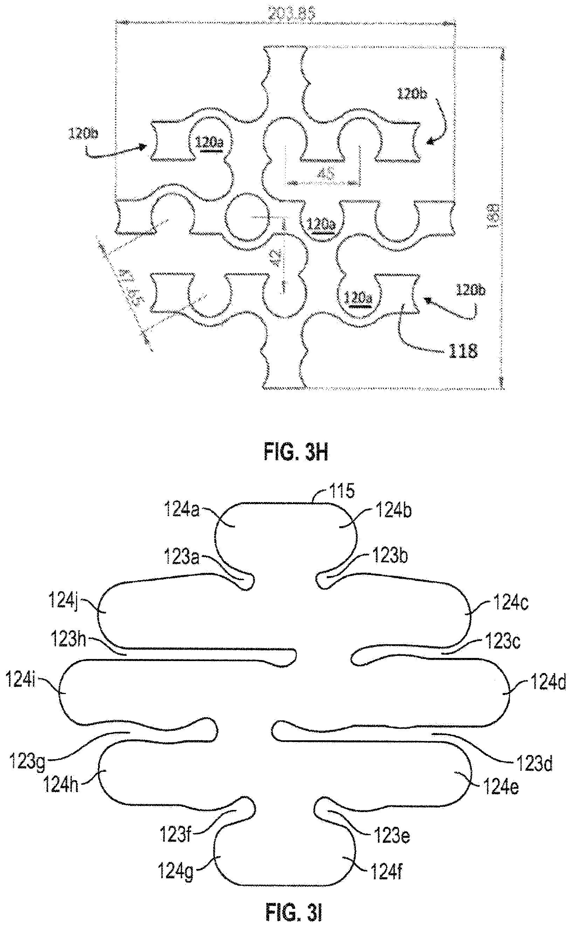

The transducer array 100 also includes a skin-level layer of adhesive 118a disposed beneath the foam layer 122, as shown in FIGS. 3A, 3H, and 3J. (The skin-level adhesive 118a also appears in FIG. 3G.) In general, the skin-level layer of adhesive 118a follows the branching configuration of the flex circuit 102 and the foam layer 122, but with the various branches and trunk portions of the skin-level adhesive 118a being slightly wider than the corresponding portions of the flex circuit 102 and the foam layer 122 so as to at least partially overlap with the spaces between the branches of the flex circuit 102 and the foam layer 122. Notably, the skin-level adhesive 118a includes cutouts 120a along the branches of the adhesive, and cutouts 120b at the free ends of the branches of the adhesive. These cutouts 120a, 120b are shaped so as not to intervene between the electrode elements 110 or the conductive gel elements 114 and the patient's skin. The skin-level layer of adhesive 118a also functions as a constructive element, to stabilize the central area around the electrode elements 110 to prevent movement of the electrode elements 110 relative to the patient's skin.

The skin-level layer of adhesive 118a may be made from a polyester/rayon-blend, spunlace non-woven tape material such as 3M^{®} 9917, which is 30 micrometers thick. The tape may be double-coated with acrylate adhesive, to provide a peel strength on the skin-facing side (e.g., 23 lbf/inch) and a higher peel strength (e.g., 27 lbf/inch) on the opposite, outer side. The material is preferably hypoallergenic, highly conformable, and breathable; with a high moisture vapor transmission rate; and it is preferably gamma sterilization-compatible. To prevent excessive sweating and moisture from being trapped under the transducer array 100, the overall surface area of the skin-level layer of adhesive 118a may be minimized, e.g., by making it just slightly wider than the corresponding portions of the flex circuit 102 and the foam layer 122.

Note that in embodiments where a conformal foam layer 122 is omitted, the layer of adhesive 118a may be connected directly to the flex circuit 102 with no intervening components disposed therebetween. Alternatively, in those embodiments where the conformal foam layer 122 is provided, the layer of adhesive 118 may be connected indirectly to the flex circuit 102, with a foam layer 122 disposed therebetween. In these embodiments, the foam layer 122 may be connected to the flex circuit 102 with a bonding material, such as an adhesive or a cohesive.

An exemplary embodiment of the support layer 115 is shown in Figs. 3I and 3J. The support layer 115 (FIGS. 3A, 3I, and 3L) is positioned below and connected to an inner side of the flex circuit 102, the inner side 111b of the electrode elements 110 or combinations thereof. The support layer 115 has a number of slots 123, which divide the support layer 115 into a number of separate fingers 124, each of which is underneath a respective branch of the flex circuit 102. The support layer 115 is provided with 8 slots 123a-h, and 10 fingers 124a-j. The slots 123a-h are preferably significantly narrower than the fingers 124a-j and the fingers 124a-j are preferably wider than the diameters of the electrode elements 110. This configuration results in the fingers 124a-j of the support layer 115 overlapping with the spaces between the branches of the flex circuit 102. The transducer array 100 may also be provided with an adhesive layer 118b positioned between the support layer 115 and the flex circuit 102 to anchor the support layer 115 to the flex circuit 102. The fingers 124a-j overlie and extend between the conductive gel elements 114. The fingers 124a-j are connected to the conductive gel elements 114 and serve to provide lateral support for the conductive gel elements 114 so as to prevent the conductive gel elements 114 from moving laterally when the conductive gel elements 114 are applied to the skin of the patient. The flexible nature of the support layer 115 allow the fingers 124a-j to move independently of each other as the branches of the flex circuit 102 move independently of each other. This, in turn, helps to maintain conformability of the transducer array 100 and adhesion to the patient's skin even as the patient moves.

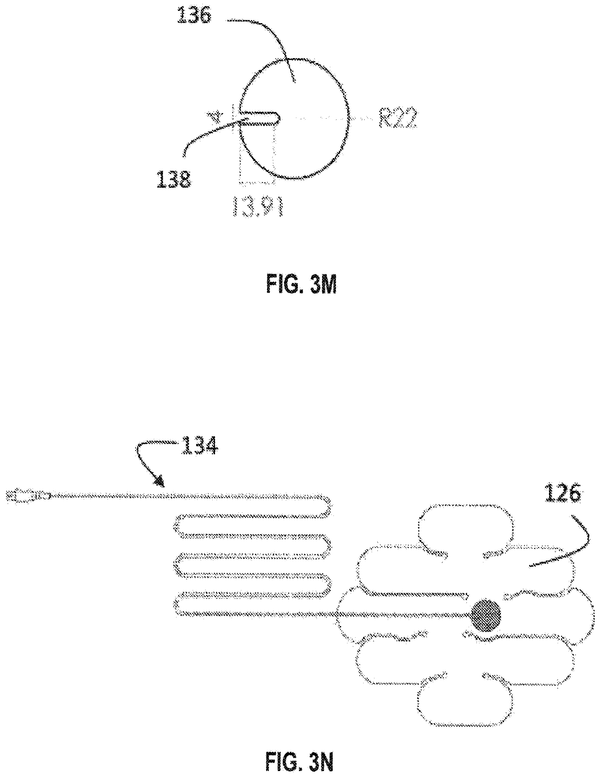

A top, covering adhesive-backed layer 126 (FIGS. 3A, 3L, and 3N) is positioned above and connected to an outer side of the flex circuit 102. The covering adhesive-backed layer 126 has a number of slots 128, which divide the covering adhesive-backed layer 126 into a number of separate fingers 130, each of which overlies a respective branch of the flex circuit 102. The slots 128 are preferably significantly narrower than the fingers 130 and the fingers 130 may be wider than the diameters of the electrode elements 110. This configuration results in the fingers 130 of the covering adhesive-backed layer 126 overlapping with the spaces between the branches of the flex circuit 102 to provide maximal adhesion of the covering adhesive-backed layer 126 to the patient's skin around the electrode elements, while still allowing the fingers 130 of the covering adhesive layer to move independently of each other as the branches of the flex circuit 102 move independently of each other. This, in turn, helps to maintain conformability of the transducer array 100 and adhesion to the patient's skin even as the patient moves. In addition, the covering adhesive-backed layer 126 preferably extends beyond the perimeter of the flex circuit 102 to provide additional adhesion to the skin at the outer boundary of the transducer array 100.

The covering adhesive-backed layer 126 may be made from 3M^{®} 9916, which is a 100% polyester, spunlace non-woven tape, for example. This material may be single-coated with acrylate adhesive on the skin-facing side, which adheres the covering adhesive-backed layer 126 to the outer surface of the flex circuit 102. The material forming the covering adhesive-backed layer 126 may have a thickness of 40 micrometers. The covering adhesive-backed layer 126 may be hypoallergenic, highly conformable, breathable, and/or gamma sterilization-compatible.

As shown in Fig. 3J, the support layer 115 may be shaped to be within the confines of the layer 126. Further, the support layer 115 may be shaped so that the fingers 124 of the support layer underlie and are aligned with fingers 130 of the layer 126 so that the fingers 124 move with the fingers 130.

Shown in Fig. 3P is a bottom plan view of a portion of the transducer assembly 100 depicting the support layer 115 adhered to the flex circuit 102, and positioned between the conductive gel element 114 and the flex circuit 102. In some embodiments, the transducer array 100 can be made by attaching the support layer 115 to the flex circuit 102 such that the electrode elements 110 are between the support layer 115 and the flex circuit 102. In this position, the conductive gel elements 114 can be applied in liquid form to the support layer 115 directly over the electrode elements 110 and then cured. In this embodiment, at least some of the liquid forming the conductive gel elements 114 may pass through the support layer 115 thereby forming the layers 106a and 106b encapsulating the support layer 115 in the conductive gel elements 114 when cured. Further, the conductive gel elements 114, when cured, may adhere to the electrode elements 110 thereby providing further lateral support to the conductive gel elements 114 separate from the adhesive layer 118b attaching the support layer 115 to the flex circuit 102. In some embodiments, the support layer 115 can be anchored to the flex circuit 102 in a manner other than an adhesive. For example, a mechanical linkage such as thread, staple or rivet can be used to connect the support layer 115 to the flex circuit 102.

Notably and advantageously, two separate factors contribute to the adhesion of the entire transducer array 100 to the patient's skin. The first factor is the portions of the lower surface of the top adhesive layer 126 that contact the skin through the spaces between the branches of the flex circuit 102 and beyond the perimeter of the flex circuit 102. The second factor is the layer of adhesive 118a disposed between the foam layer 122 and the person's skin (or, between the flex circuit 102 and the person's skin in those embodiments that do not include the foam layer 122). The inclusion of these two separate adhesive components provides significantly improve adhesion of the transducer array 100 to the patient's skin. This feature of the transducer array 100 enhances the degree of adhesion of the transducer array 100 to the patient's skin around the electrode elements, resulting in prolonged and better skin/electrode contact as compared to configurations in which the only adhesion was provided by an adhesive-backed patch overlying the entire transducer array.

In some embodiments, the covering adhesive-backed layer 126 includes a central aperture 135 and a slit 132 extending from the innermost end 129 of one of the slots 128 - in particular, the innermost slit-end that is closest to the central aperture 135. The central aperture 135 permits an electrical cable 134 (shown in FIG. 3N) that protrudes from the back surface of the flex circuit 102 to extend through the covering adhesive-backed layer 126. This electrical cable 134 is used to connect the flex circuit 102 to a TTFields therapy controller (not illustrated) via a connector. The slit 132 is useful for positioning the adhesive-backed layer 126 over the flex circuit 102 after the cable 134 has been connected to the flex circuit 102 during the assembly process. In particular, portions of the covering adhesive-backed layer 126 can be moved away from each other to open the slit 132, such that the covering adhesive-backed layer 126 can be passed around the electrical cable 134 on either side and then the entire adhesive-backed layer can be pressed into proper position.

Once the transducer array 100 has been properly attached to the patient's skin with the covering adhesive-backed layer 126 securing the transducer array 100 in place, the central aperture 135 may be covered, for protection, with a top adhesive-backed slot-cover 136 (FIGS. 3A, 3M, and 3N). The slot-cover 136 may be a disc-shaped item, formed from the same material and in the same manner as the covering adhesive-backed layer 126. In some preferred embodiments, the slot cover 136 includes a slot 138 for the electric cable 134 to pass through.

In some embodiments, the entire assembly of components described above is protected, prior to use on a patient, with a two-part release liner 140 (FIGS. 3A and 3O). The release liner 140 has an overall shape that generally follows, but may be slightly larger than, the outer periphery of the covering adhesive-backed layer 126. It may be made from, for example, AR W4000, available from Adhesive Research, which is a white, silicone-coated PET (polyethylene terephthalate) material that is 50 micrometers thick.

In the FIGS. 3A-3O embodiment of a transducer array 100 described above, there are 20 electrode elements arranged in five rows, with two, five, six, five, and two electrode elements in each of the successive rows. (The rows correspond with the branches of the flex circuit 102 and are perpendicular to the longitudinal direction in which the trunk 108 extends. Thus, the rows are oriented horizontally and the trunk 108 is oriented vertically as the transducer array 100 is oriented in FIG. 1 and the flex circuit 102 is oriented in FIG. 2.) Depending on factors such as the size, sex, age, etc. of a patient, however, there could be more or less electrode elements 110 arranged in different configurations, while still adhering to the inventive concepts disclosed herein.

In both the FIGS. 3A-3P embodiment, the flex circuit 102 has a plurality of branches extending on each lateral side of the trunk region. But in alternative embodiments, the branches may be present only on a single lateral side of the trunk region (in which case, the trunk region would be located near one edge of the apparatus.

In some embodiments (including but not limited to the FIGS. 3A-3P embodiment) the flex circuit 102 is configured so that no more than three paths emanate from any given intersection on the flex circuit 102. For example, in FIG. 3B, one path of the flex circuit 102 emanates from the intersections at the mounting pads 104a, two paths of the flex circuit 102 emanate from the intersections at the mounting pads 104b, and three paths of the flex circuit emanate from the intersections at the mounting pads 104c. Notably, there are no intersections on the flex circuit 102 from which more than three paths emanate. This holds true for both the intersections that are positioned at the mounting pads 104, and also for intersections that are not positioned at one of the mounting pads 104 (e.g., The T-shaped intersections 105).

In some embodiments, including the FIGS. 3A-3P embodiments, all segments of the flex circuit 102 are straight.

In some embodiments, including the FIGS. 3A-3P embodiments, an electrical cable terminates on the flex circuit 102 (as best seen in FIG. 3N). Optionally, in these embodiments, (as best seen in FIG. 3B) segments of the flex circuit 102 near the distal end of each branch are thinner than at least some of the segments of the flex circuit 102 that are adjacent to the location where the electrical cable terminates (e.g., segment 108d). This configuration increases the flexibility of the flex circuit, which also contributes to improving the flexibility of the entire apparatus.

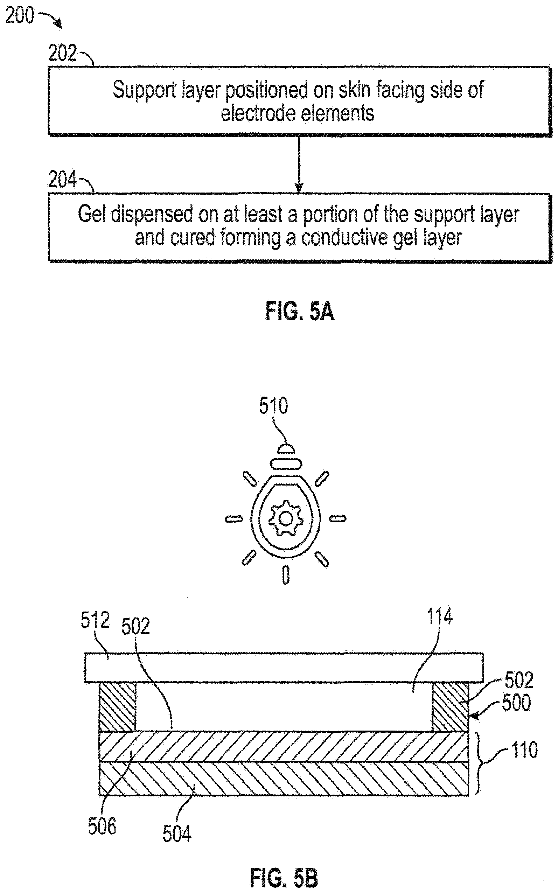

FIG. 5A illustrates a flow chart 200 of an exemplary method of forming the exemplary conductive gel elements 114 in accordance with the present disclosure. In a step 204, the support layer 115 may be positioned on and optionally anchored to the electrode elements 110 and the flex circuit 102. In a step 206, conductive gel (in liquid form) or semi-solid conductive gel may be dispensed in a predetermined pattern on at least a portion of the support layer 115 and cured (e.g., UV curing, electron beam curing) to form the conductive gel elements 114. In forming the conductive gel elements 114, the conductive gel or semi-solid conductive gel may be dispensed at one or more pre-determined targeted locations onto the electrode elements 110. The electrode elements 110 may be provided in a predetermined pattern, and have a predetermined size. In these embodiments, the pre-determined targeted locations where conductive gel will be dispensed may correspond to the predetermined pattern and predetermined size of the electrode elements 110 so that when the conductive gel elements 114 are installed on the electrode elements 110, the conductive gel elements 114 each correspond to at least one electrode element 110.

In some embodiments, a mold and/or spacer may be used to provide for dispensing of the conductive gel (in liquid form) or semi-solid conductive gel at the one or more pre-determined targeted locations. For example, FIG. 5B illustrates an exemplary mold 500 disposed on a surface 502 of the electrode element 110. The electrode element 110 includes a conductive material layer 504 and a polymer layer 506. Generally, the mold 500 may be positioned prior to applying conductive gel element 114 (in liquid form) thereto. A sidewall 508 of the mold 500 may extend beyond the electrode element 110 and define a thickness for the semi-solid conductive gel element 114. Alternatively, the mold 500 may be sized and shaped to accept both the electrode element 110 and the conductive gel element 114. In this example, the electrode element 110 may be first disposed within the mold 500, with the mold 500 having a sidewall height greater than height of the electrode element 110, such that a portion of the sidewall 508 of the mold 500 that extends beyond the electrode element 110 defines a thickness for the semi-solid conductive gel element 114 produced thereon. The conductive gel element 114 may be then cured via a radiation source 510 (e.g., UV curing) such that the mold 500 provides boundaries and wall height equal to a pre-determined thickness desired for the conductive gel element 114. The curing provides the polymerized conductive gel element 114. In some embodiments, the conductive gel elements 114 may have a surface area that overlaps about 75% to 100% of the surface area of the particular electrode element 110. In some embodiments, the conductive gel elements 114 may have a surface area that overlaps about 50% to 100% of the surface area of the particular electrode element 110.

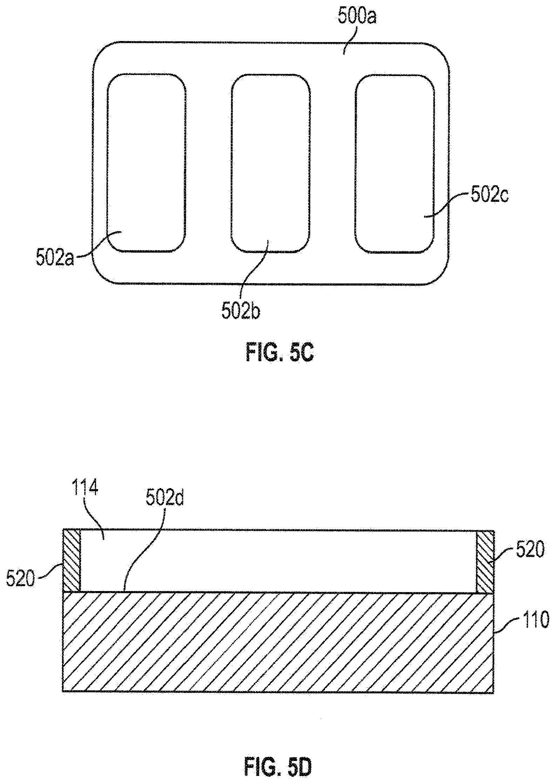

Curing the conductive gel element 114 directly on the electrode element 110 allows for in-line processing. FIG. 5C depicts in-line processing of multiple elements 110 or arrays 100 using a mold 500a that contacts first surfaces 502a, 502b and 502c of three separate electrode elements 110. It should be noted that production for any number of elements 110 or arrays in batch or continuous format is contemplated.

In some embodiments, a quartz plate 512 (see FIG. 5B) may optionally be used during curing. The quartz plate 512 may be disposed on the liquid or semi-solid conductive gel element 114 applied to the surface 502 of the electrode element 110 prior to curing. The quartz plate 512 may be retained on the conductive gel element 114 during a portion or all of the curing step. The optional use of the quartz plate 512 may transfer radiation (e.g., UV light) therethrough providing homogeneous thickness of the polymerized conductive gel element 114.

Referring to FIG. 5D, in some embodiments, one or more barriers 520 may be applied to the surface 502d of the electrode element 110 to maintain the conductive gel element 114 on the electrode element 110. The barrier 520 may be formed of any material configured to be attached to the surface 502d of the electrode element 110 and maintain a perimeter sidewall and receiving space for the conductive gel element 114. When the barrier 520 is formed of a polymer or other porous material (such as, but not limited to, MED 5695R, available from Vancive Medical Technologies, a polyethylene foam), the conductive gel element 114 may penetrate into the barrier 520 prior to curing and increase adhesion between the conductive gel element 114 and the electrode element 110. Further, when the barrier 520 is formed of a polymer, the conductive gel element 114 may crosslink to the polymer of the barrier 520 during curing and yet further increase the adhesion between the conductive gel element 114 and the electrode element 110. Barriers 520 may be similar to hydrogel barriers 116 shown in FIGS. 3A, 3F, 3G and 3J.

The barrier 520 can be utilized with electrodes of any structure/configuration and produced from any material(s) as described herein. The use of hydrogel barriers can be particularly advantageous when a ceramic electrode is utilized (or when at least the surface on which the liquid hydrogel is disposed is formed of a ceramic material), as the hydrogel cannot crosslink to the ceramic; in this embodiment, the hydrogel barrier serves as to anchor the hydrogel to the electrode and prevent migration of the hydrogel from its correct location on the electrode.

In some embodiments, the electric field generator 32, connected to the transducer array 100, may supply a first electric signal having a first power and a first frequency to a first group of one or more electrode elements 110 at a first instance in time to generate a first TTField. The electric field generator 32, at a second instance in time, may supply a second electric signal having a second power, the same as or different from the first power, and a second frequency, the same as or different from the first frequency, to a second group of electrode elements 110 to generate a second TTField. The first TTField and the second TTField may target the same target area or may target different target areas. In one embodiment, the first instance in time and the second instance in time may overlap, that is, the electric field generator 32 may supply the second electric signal to the second group while also supplying the first electric signal to the first group. In such an embodiment, the first group and the second group may be mutually exclusive.

In some embodiments, the electric field generator 32, connected to the transducer array 100, may supply a first electrical signal having a first power and a first frequency to a first group of one or more electrode element 110 and supply a second electrical signal having a second power and a second frequency to a second group of electrode elements 110 at the same instance in time. That is, the electric field generator 32 may simultaneously supply the first electric signal to the first group and the second electric signal to the second group. While the above embodiments describe only the first group and the second group, it is understood that there may be more than two groups. In one embodiment, the number of groups is dependent on the number of combinations of the conductive regions 56a-h.

Referring again to FIG. 2, in some embodiments, leads 34a and 34b may include a DC blocking component, such as blocking capacitors 160a and 160b. Blocking capacitors may be operable to block DC current from passing to the pads 42a and 42b. The blocking capacitors 160a and 160b pass AC voltage to the pads 42a and 42b, and may be operable to prevent DC voltage or DC offset generated by the electric field generator 32 or otherwise present in the electrical signal from passing to or through the patient. In some embodiments, the blocking capacitors 160a and 160b may be non-polarized capacitors. In some embodiments, the blocking capacitors 160a and 160b may have a capacitance of about 1µF. In some embodiments, the blocking capacitor is a "Goldmax, 300 Series, Conformally Coated, X7R Dielectric, 25-250 VDC (Commercial Grade)" leaded non-polarized ceramic capacitor by KEMET Electronics Corporation (Fort Lauderdale, FL).

In some embodiments, the blocking capacitors 160a and 160b may be a component of the leads 34a and 34b, or an additional component at any position between the electrode element 82 of the first pad 42a and second pad 42b and the electric field generator 32. For example, the blocking capacitors 160a and 160b may be intermediate the first end 36a of the second conductive lead 34b and the electric field generator 32, or intermediate the second end 40b of the second conductive lead 34b and the second pad 42b. In some embodiments, one or more blocking capacitor 160a and 160b may be provided remote from the pads 42a and 42b. For example, one or more blocking capacitors 160a and 160b may be located on a non-patient side of the electrode element 82

Certain non-limiting embodiments of the present disclosure are related to kits that include components of the TTField generating systems, such as the electronic apparatus 30, described herein. In some embodiments, one or more of the pad 42a and 42b, or transducer array 100 may be packaged as part of a kit. In some embodiments, the kit may include the first pad 42a and the lead 34a connected to the electrode elements 110. In some embodiments, the kit may include the first pad(s) 42a and second pad(s) 42b, the transducer array 100 and the leads 34a and 34b. In some embodiments, the lead 34a may be mechanically coupled to the first pad 42a, and the second conductive lead 34b may be mechanically coupled to the second pad 42b, for example, by a rivet, by solder, by adhesive, by welding, and/or other electrically conductive coupling means. In some embodiments, the kit may further include the blocking capacitor(s) 160a or 160b positioned such that the electric signal passes through the blocking capacitor 160a or 160b.

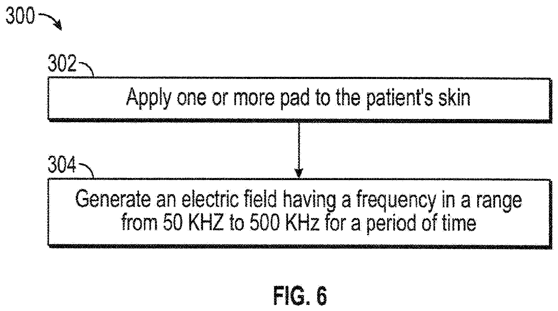

Referring now to FIG. 6, shown therein is a flow chart 300 of an exemplary method of using the electronic apparatus 30 and the transducer array 100 to apply a TTField to a patient. In a step 302, the transducer array 100 may be attached to the skin of a patient. For example, electrode elements 110 may be attached to the skin of the patient on opposite sides of a tumor. In the context of a brain tumor, electrodes 110 may be positioned in the center of a person's head. For example, one of the electrode elements 110 may be positioned on the right side of the person's head, and another one of the electrode elements 110 may be positioned on the left side of the person's head. One or more electrode elements 110 may be applied to the patient's skin by a user.

In a step 304, an AC voltage is applied between the electrode elements 110. For example, the electric field generator 32 provides an alternating electric field having a frequency in a range of from about 50 kHz to about 1 MHz for a period of time to the electrode elements 110 applied to the patient to deliver TTF fields to the patient. In some embodiments, a user may initiate generation of the electric field generator 32 via the control box 28. In some embodiments, application of the AC voltage may be performed more than one time in the period of time. Duration of multiple instances of application of AC voltage may be similar or different. In some embodiments, a time period of non-application of AC voltage may be between application of AC voltage.

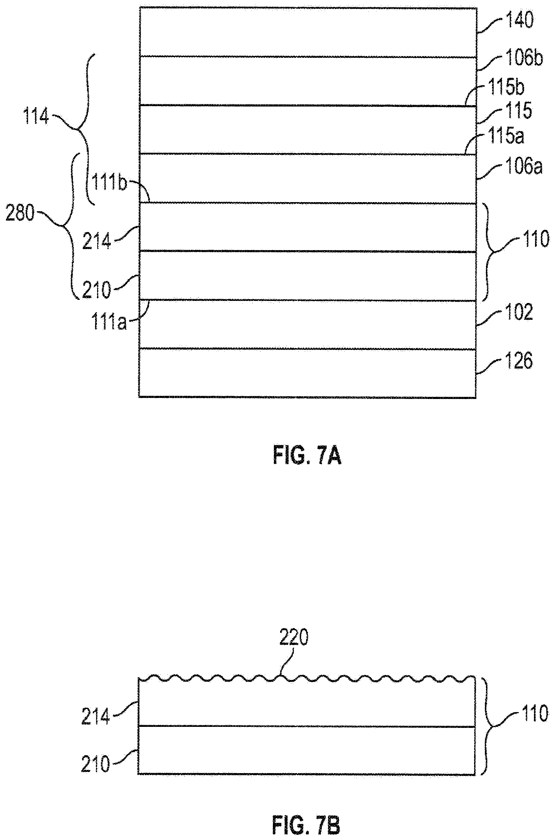

FIG. 7A is a partial schematic view of the transducer array 100 constructed in accordance with the present disclosure. FIG. 7A is a cross-sectional view taken across one of the electrode elements 110. The electrode element 110 includes a conductive plate 210 and a dielectric material 214 positioned adjacent to and covering the conductive plate 210. The conductive plate 210 is constructed of a conductive material, such as copper, aluminum or the like. The dielectric material 214 is constructed of a non-conductive material, such as a ceramic material, a polymer material or the like.

The transducer array 100 also includes a plurality of isolated conductive gel elements 114, one of which is shown by way of example in Fig. 7A. The isolated conductive gel element 114 is in contact with the electrode element 110. The dielectric material 214 is positioned between the isolated conductive gel element 114, and serves to capacitively couple the conductive plate 210, and the isolated conductive gel element 114. The isolated conductive gel element 114 includes the first conductive gel layer 106a and the second conductive gel layer 106b. The support layer 115 is optional, and when present may be positioned in between the first conductive gel layer 106a in the second conductive gel layer 106b. As shown in Fig. 7A, the first conductive gel layer 106a overlaps the second conductive gel layer 106b. As will be explained in more detail below, the first conductive gel layer 106a may be applied to the plurality of electrode elements 110 in a flowable state, and then cured on the electrode elements 110. The first side 115a of the support layer 115 may be applied onto the first conductive gel layer 106a, and the second conductive gel layer 106b, in a flowable state, may be applied onto the second side 115b of the support layer 115 such that the first and second conductive gel layers 106a and 106b on each of the electrode elements 110 overlap. Once the second conductive gel layer 106b is applied, the second conductive gel layer 106b may be cured on the second side 115b of the support layer 115. As discussed above, the support layer 115 may be provided with a plurality of pores which permit the second conductive gel layer 106b to flow through the pores and engage the first conductive gel layer 106a prior to curing the second conductive gel layer 106b.

The release liner 140 is in contact with and covers the second conductive gel layer 106b. The release liner 140 may be applied to the second conductive gel layer 106b subsequent to curing the second conductive gel layer 106b.

The first conductive gel layer 106a may have a thickness of within a range of approximately 0.1mm to approximately 4mm and combinations therein (e.g, a range of from 0.1 mm to about 1.4mm, a range of from about .2mm to about 3mm, etc.). The second conductive gel layer 106b may have a thickness within a range of approximately 0.1mm to approximately 4 mm and combinations therein (e.g., a range of from 0.1 mm to about 1.4mm, a range of from about .2mm to about 3mm, etc.).

Any dielectric polymer material(s) known in the art or otherwise contemplated herein may be present in the electrodes utilized in accordance with the present disclosure. Non-limiting examples of polymers that may be utilized to form the electrode (and in particular, to form a polymer layer of an electrode) include PVDF, poly(vinylidene fluoride-trifluoroethylene-chlorotrifluoroethylene), and/or poly(vinylidene fluoride-trifluoroethylene-1-chlorofluoroethylene). Those two polymers are abbreviated herein as "Poly(VDF-TrFE-CtFE)" and "Poly(VDF-TrFE-CFE)," respectively. These polymers have a high dielectric constant (i.e., on the order of 40). Alternatively, other polymer(s) that provides a high level of capacitance (i.e., a dielectric constant of at least 20 at least one frequency between 100 kHz and 500 kHz) may be used.

In addition, in certain non-limiting embodiments, ceramic nanoparticles may be mixed into the polymer to form a "nanocomposite." Optionally, these ceramic nanoparticles may comprise ferroelectric metal oxides (e.g., at least one of barium titanate and barium strontium titanate).

When the electrode element 110 comprises a conductive material layer and a flexible polymer layer, the layer of conductive material may comprise at least one metal (such as, but not limited to, stainless steel, gold, and/or copper).

When the dielectric material 214 is constructed of a ceramic material, the ceramic material may be porous. When the first conductive gel layer 106a is applied to the dielectric material 214, a portion of the first conductive gel layer 106a in the flowable state may flow into the pores and thereby penetrate the dielectric material prior to curing the first conductive gel layer 106a. Once cured, the portions of the first conductive gel layer 106a penetrating the dielectric material 214 enhance the adhesion of the first conductive gel layer 106a to the dielectric material 214.

When the dielectric material 214 is constructed of a non-ceramic material, such as a flexible or non-flexible polymer material, a surface of the polymer material may be treated to enhance adhesion between the first conductive gel layer 106a and the dielectric material 214. The use of a polymer in the production of the electrode element 110 may provide crosslinking between the conductive gel element 114 and the polymer of the electrode element 110 during a curing step. Chemical bonding may provide a mechanical connection therebetween that cannot be achieved with electrodes formed of only ceramic materials. Such bonding may aid in maintaining of the conductive gel element 114 on the array for a longer period of time, improved adhesion rate for a longer period of time, improved contact with skin of a patient, reduction of replacement rate of arrays, and/or the like. Examples of manners to treat the polymer material will be described below.

As shown in Fig. 7A, the electrode element 110 is connected to and supported by the flex circuit 102. As shown, the electrode element 110 extends over a portion of the flex circuit 102. The flex circuit 102 is connected to the covering adhesive backed layer 126.

Referring now to Fig. 7B, shown therein is the electrode element 110. The electrode element 110 is provided with the conductive plate 210 and the dielectric material 214 as described above. In the example shown in Fig. 7B, the dielectric material 214 is constructed of a non-porous material, such as a polymer. To enhance adhesion with the conductive gel layer 106a, the dielectric material 214 is provided with a textured surface 220. The textured surface 220 can be formed by any suitable process, such as abrasion, chemical etching, blasting, grinding, plasma treatment, ozone treatment, and/or the like. The textured surface 220 is spaced a distance away from the conductive plate 210.

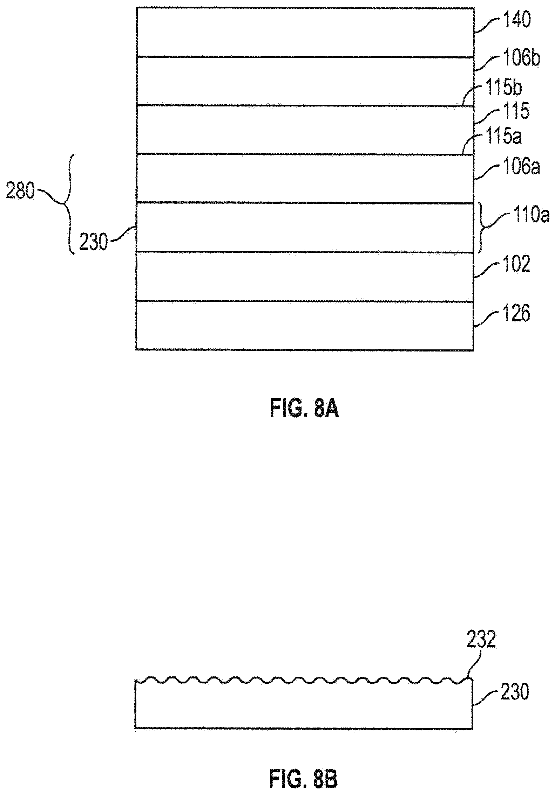

Referring now to Fig. 8A, shown therein is a partial schematic view of another embodiment of a transducer array 100a constructed in accordance with the present disclosure. The transducer array 100a is identical in construction and function as the transducer array 100 discussed above, except that the transducer array 100a is provided with the plurality of electrode elements 110a. FIG. 8A is a cross-sectional view taken across one of the electrode elements 110a. The electrode element 110a is provided with a conductive layer 230, which is mechanically and electrically connected to the first conductive gel layer 106a.

Referring now to Fig. 8B, shown therein is the electrode element 110a. The electrode element 110a is provided with the conductive layer 230 as described above. To enhance adhesion with the conductive gel layer 106a, the conductive layer 230 is provided with a textured surface 232. The textured surface 232 can be formed by any suitable process, such as abrasion, chemical etching, blasting, grinding, plasma treatment, ozone treatment, and/or the like.

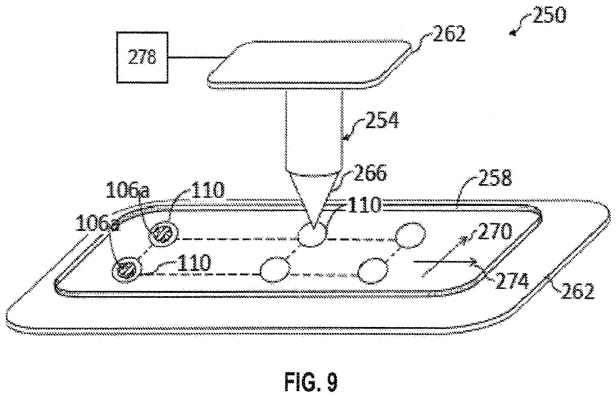

Referring now to FIG. 9, shown therein is a diagram of an exemplary embodiment of a gel application system 250 constructed in accordance with the present disclosure that applies the first conductive gel layer 106a and/or the second conductive gel layer 106b to the electrode element 110 or 110a. The gel application system 250 comprises one or more applicator 254 and a platform 258 moveably attached to a housing 262. Only one applicator 254 is shown for purposes of brevity. It should be understood that multiple applicators 254 can also be used. The one or more applicator 254 further comprises at least a nozzle 266 to eject a conductive gel, described in more detail above. The platform 258 supports a plurality of electrode elements 110 or 110a of the transducer arrays 100 or 100a. The plurality of electrode elements 110 or 110a may be connected to and supported by the flex circuit 102 and/or the cover adhesive-backed layer 126 in the first predetermined pattern discussed above. The cover adhesive-backed layer 126 may engage and be supported by the platform 258. In one embodiment, the applicator 254 may move in one of a first direction 270 or a second direction 274 or a combination of the first direction 270 and the second direction 274. In one embodiment, the platform 258 may move in one of the first direction 270 or the second direction 274 or a combination of the first direction 270 and the second direction 274. In one embodiment, the gel application system 250 includes a controller 278 to control movement of the platform 258 and/or to control movement of the applicator 254.

In one embodiment, the nozzle 266 has an application distance determined by the distance between the nozzle 266 from the platform 258, and ejects conductive gel (in liquid form) at an application pressure, and moves at an application velocity relative to the platform 258. By adjusting the application distance, the application pressure, and the application velocity, the amount of conductive gel applied by the nozzle 266 can be adjusted. The application velocity may be caused by moving the applicator 254 and/or the platform 258 in one of the first direction 270, the second direction 274, or the combination of the first direction 270 and the second direction 274.

In one embodiment, the application pressure is selected such that a portion of the conductive gel is wicked into pores of the dielectric material 214 when the dielectric material 214 is porous, or wicked into the valleys 224 or 236 so that a contact area, that is an area of the electrode element 110 or 110a and the first conductive gel layer 106a in contact, is increased. For example, it may be desirable to eject conductive gel at a higher pressure to cause the conductive gel to penetrate further into the ceramic material. By increasing penetration into the ceramic material, adhesion between the electrode element 110 and the first conductive gel layer 106a may be increased.

In one embodiment, the gel application system 250 ejects conductive gel in a liquid form (e.g., a flowable state) onto the textured surface 220 or 232. Once the gel application system 250 ejects conductive gel onto a particular electrode element 110 or 110a, the gel application system 250 may eject conductive gel onto another one or more of the electrode elements 110 or 110a until the conductive gel has been applied to all of the electrode elements 110 or 110a. Once the conductive gel is applied, the conductive gel is cured on the electrode elements 110 or 110a. For example, the liquid conductive gel may be exposed to a UV light emitted by a UV source to cure the liquid conductive gel into a non-flowable state, e.g., polymerized. The polymerized conductive gel may form the first conductive gel layer 106a.

In one embodiment, the applicator 254 may be hand-held, that is, the applicator 254 may be held and/or moved by a user instead of being moveably attached to the housing 262. In such an embodiment, the user may use the applicator 254 to eject conductive gel onto the dielectric material 214 of the conductive layer 230.

Once the first conductive gel layer 106a is formed, the support layer 115 may be applied to the electrode elements 110 or 110a, and then the applicator 254 may be used to apply conductive gel onto the support layer 115 to form the second conductive gel layer 106b. As discussed above, the support layer 115 is optional and therefore applying the support layer 115 to the first conductive gel layer 106a is also optional.

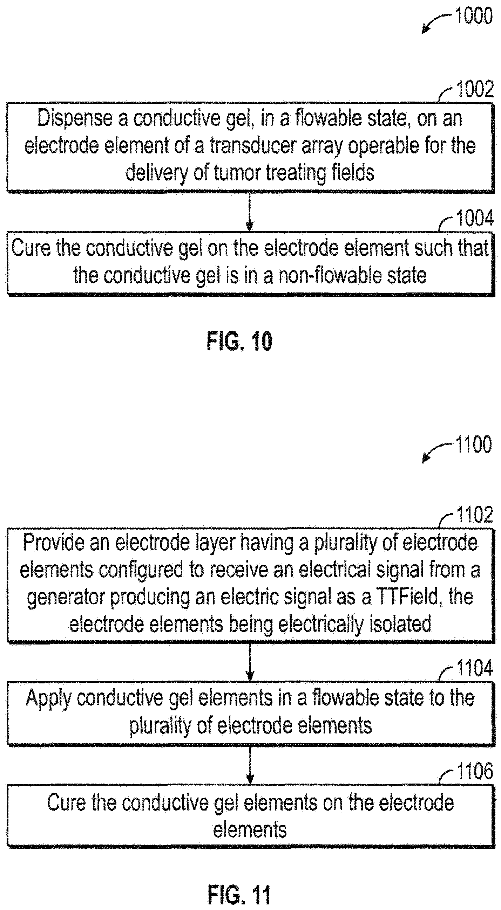

Shown in Fig. 10 is a process 1000 for making at least one tumor treating field electrode 280 (see Fig. 7A and Fig. 7B). The tumor treating field electrode 280 is a conductive gel assembly. More particularly, at a step 1002, a conductive gel is dispensed, in a flowable state, on one of the electrode elements 110 or 110a of the transducer array 100 or 100a operable for the delivery of tumor treating fields. Then, at a step 1004, the conductive gel is cured on the electrode element 110 or 110a such that the conductive gel is in a non-flowable state.

Shown in Fig. 11 is a process 1100 for making the transducer array 100 or 100a. In a step 1102, an electrode layer having a plurality of electrode elements 110 or 110a configured to receive an electrical signal from an electric field generator producing an electric signal as a TTField is provided. The electrode elements 110 or 110a are electrically isolated. In a step 1104, conductive gel elements 114 are applied in a flowable state to the plurality of electrode elements 110 or 110a. In a step 1106, the conductive gel elements are cured on the electrode elements.

From the above description, it is dear that the inventive concepts disclosed and claimed herein are well adapted to carry out the objects and to attain the advantages mentioned herein, as well as those inherent in the invention. While exemplary embodiments of the inventive concepts have been described for purposes of this disclosure, it will be understood that numerous changes may be made which will readily suggest themselves to those skilled in the art and which are accomplished within the scope of the invention as claimed herein.

## Claims

1. A transducer array, comprising:
a support layer (115) having first and second sides (115a, b);
a plurality of electrode elements (110; 110a) configured to receive an electrical signal from a generator producing an electric signal as a tumor treating field, TTField; and
a plurality of conductive gel elements (114) including one or more conductive gel layers (106; 106a, b) and corresponding to respective ones of the electrode elements (110; 110a);
wherein the conductive gel elements (114) are attached to one side (115a, b) of the support layer (115) and thereby anchored to the support layer (115) to reduce lateral movement of the conductive gel elements (114) in relation to the electrode elements (110; 110a).

2. The transducer array of claim 1, wherein the support layer (115) is a flexible material.

3. The transducer array of claim 1, wherein the support layer (115) is formed of a non-conductive material.

4. The transducer array of claim 1, wherein at least a portion of or the entire support layer (115) is a non-metal conductive material, optionally carbon.

5. The transducer array of claim 1, wherein the support layer (115) is formed of a woven or non-woven material, optionally a non-woven mesh.

6. The transducer array of claim 1, wherein at least a portion of the support layer (115) includes Reemay (spun nylon).

7. The transducer array of claim 1, wherein the support layer (115) is provided outside of the conductive gel elements (114).

8. The transducer array of claim 1, wherein the support layer (115) is provided outside of the conductive gel elements (114), with the electrode elements (110; 110a) connected to the first side (115a) of the support layer (115) and the conductive gel elements (114) on the second side (115b) of the support layer (115).

9. The transducer array of claim 1, wherein the support layer (115) is provided within the conductive gel elements (114).

10. The transducer array of claim 1, wherein the support layer (115) is a flexible material having a plurality of voids intersecting the first and second sides (115a, 115b) thereof and provided within the conductive gel elements (114), and the conductive gel elements (114) comprise a first conductive gel layer (106a) on the first side (115a) of the support layer (115) and a second conductive gel layer (106b) on the second side (115b) of the support layer (115), with each of the second conductive gel layers (106b) overlapping the corresponding first conductive gel layers (106a).

11. The transducer array of claim 10, wherein the first and second conductive gel layers (106a, b) are constructed of different conductive gels.

12. The transducer array of claim 10, wherein the first and second conductive gel layers (106a, b) are constructed of the same conductive gel.

13. The transducer array of claim 1, wherein the conductive gel elements (114) comprise discs slightly larger in diameter than the electrode elements (110; 110a).

14. The transducer array of claim 1, wherein the electrode elements (110; 110a) are electrically isolated.

15. The transducer array of claim 1, wherein each of the electrode elements (110; 110a) is coated with a dielectric material selected from a ceramic material, a non-flexible polymer or a flexible polymer film.

## Patentansprüche

1. Ein Wandlerarray, umfassend:
eine Trägerschicht (115), die erste und zweite Seiten (115a, b) beinhaltet;
eine Vielzahl von Elektrodenelementen (110; 110a), die so konfiguriert sind, dass sie ein elektrisches Signal von einem Generator empfangen, der ein elektrisches Signal als ein Tumortherapiefeld, TTField produziert; und
eine Vielzahl von leitfähigen Gelelementen (114), die eine oder mehrere leitfähige Gelschichten (106; 106a, b) umfassen und entsprechenden Elektrodenelementen (110; 110a) entspricht;
wobei die leitfähigen Gelelemente (114) an einer Seite (115a, b) der Trägerschicht (115) befestigt werden und dadurch mit der Trägerschicht (115) verankert sind, um seitliche Bewegungen der leitfähigen Gelelemente (114) in Bezug zu den Elektrodenelementen (110; 110a) zu verhindern.

2. Der Wandlerarray nach Anspruch 1, wobei die Trägerschicht (115) ein flexibles Material ist.

3. Der Wandlerarray nach Anspruch 1, wobei die Trägerschicht (115) aus einem nichtleitenden Material gebildet ist.

4. Der Wandlerarray nach Anspruch 1, wobei mindestens ein Teil oder die gesamte Trägerschicht (115) ein nichtleitendes Material, gegebenenfalls Kohlenstoff, ist.

5. Der Wandlerarray nach Anspruch 1, wobei die Trägerschicht (115) aus einem gewebten oder nicht gewebten Material, gegebenenfalls ein nicht gewebtes Netz, gebildet ist.

6. Der Wandlerarray nach Anspruch 1, wobei mindestens ein Teil der Trägerschicht (115) Reemay (gesponnenes Nylon) enthält.

7. Der Wandlerarray nach Anspruch 1, wobei die Trägerschicht (115) außerhalb der leitfähigen Gelelemente (114) angebracht ist.

8. Der Wandlerarray nach Anspruch 1, wobei die Trägerschicht (115) außerhalb der leitfähigen Gelelementen (114) angeordnet ist, wobei die Elektrodenelemente (110; 110a) mit der ersten Seite (115a) der Trägerschicht (115) verbunden sind und sich die leitfähigen Gelelementen (114) auf der zweiten Seite (115b) der Trägerschicht (115) befinden.

9. Der Wandlerarray nach Anspruch 1, wobei die Trägerschicht (115) innerhalb der leitfähigen Gelelemente (114) angebracht ist.

10. Der Wandlerarray nach Anspruch 1, wobei die Trägerschicht (115) ein flexibles Material ist, das eine Vielzahl von Hohlräumen aufweist, welche sich mit den ersten und zweiten Seiten (115a, 115b) davon schneiden, und in den leitfähigen Gelelementen (114) enthalten ist, wobei die leitfähigen Gelelemente (114) eine erste leitfähige Gelschicht (106a) auf der ersten Seite (115a) der Trägerschicht (115) und eine zweite leitfähige Gelschicht (106b) auf der zweiten Seite (115a) der Trägerschicht (115) umfassen, wobei jede der zweiten leitfähigen Gelschichten (106b) eine entsprechende erste leitfähige Gelschicht (106a) überlappt.

11. Der Wandlerarray nach Anspruch 10, wobei die erste und zweite leitfähige Gelschicht (106a, b) aus unterschiedlichen leitfähigen Gelen hergestellt sind.

12. Der Wandlerarray nach Anspruch 10, wobei die erste und zweite leitfähige Gelschicht (106a, b) aus demselben leitfähigen Gel hergestellt sind.

13. Der Wandlerarray nach Anspruch 1, wobei die leitfähigen Gelelemente (114) leicht größere Scheiben in Diameter als die Elektrodenelemente (110; 110a) umfassen.

14. Der Wandlerarray nach Anspruch 1, wobei die Elektrodenelemente (110; 110a) elektrisch isoliert sind.

15. Der Wandlerarray nach Anspruch 1, wobei jedes der Elektrodenelemente (110; 110a) mit einem von Keramikmaterial ausgewählten dielektrischen Material, einem nicht-flexiblen Polymer oder flexiblen Polymerfilm beschichtet ist.

## Revendications

1. Réseau de transducteurs, comprenant :
une couche de support (115) ayant des première et deuxième côtés (115a, b) ;
une pluralité d'éléments d'électrode (110 ; 110a) conçus pour recevoir un signal électrique provenant d'un générateur produisant un signal électrique sous forme de champ de traitement de la tumeur, TTField ; et
une pluralité d'éléments de gel conducteur (114) comprenant une ou plusieurs couches de gel conducteur (106 ; 106a, b) et correspondant aux éléments d'électrode respectifs (110 ; 110a) ;
dans lequel les éléments de gel conducteur (114) sont fixés à un côté (115a, b) de la couche de support (115) et ainsi ancrés à la couche de support (115) pour réduire le mouvement latéral des éléments de gel conducteur (114) par rapport aux éléments d'électrode (110 ; 110a).

2. Réseau de transducteurs selon la revendication 1, dans lequel la couche de support (115) est un matériau flexible.

3. Réseau de transducteurs selon la revendication 1, dans lequel la couche de support (115) est faite d'un matériau non conducteur.

4. Réseau de transducteurs selon la revendication 1, dans lequel au moins une partie ou la totalité de la couche de support (115) est un matériau conducteur non métallique, éventuellement du carbone.

5. Réseau de transducteurs selon la revendication 1, dans lequel la couche de support (115) est faite d'un matériau tissé ou non tissé, éventuellement d'un maillage non tissé.

6. Réseau de transducteurs selon la revendication 1, dans lequel au moins une partie de la couche de support (115) comprend du Reemay (nylon filé).

7. Réseau de transducteurs selon la revendication 1, dans lequel la couche de support (115) est prévue à l'extérieur des éléments de gel conducteurs (114).

8. Réseau de transducteurs selon la revendication 1, dans lequel la couche de support (115) est prévue à l'extérieur des éléments de gel conducteurs (114), les éléments d'électrode (110 ; 110a) étant connectés au premier côté (115a) de la couche de support (115) et les éléments de gel conducteurs (114) au deuxième côté (115b) de la couche de support (115).

9. Réseau de transducteurs selon la revendication 1, dans lequel la couche de support (115) est prévue à l'intérieur des éléments de gel conducteur (114).

10. Réseau de transducteurs selon la revendication 1, dans lequel la couche de support (115) est un matériau flexible comportant une pluralité de vides qui croisent ses premier et deuxième côtés (115a, 115b) et est prévue à l'intérieur des éléments de gel conducteur (114), et les éléments de gel conducteur (114) comprennent une première couche de gel conducteur (106a) sur le premier côté (115a) de la couche de support (115) et une deuxième couche de gel conducteur (106b) sur le deuxième côté (115b) de la couche de support (115), chacune des deuxièmes couches de gel conducteur (106b) chevauchant les premières couches de gel conducteur correspondantes (106a).

11. Réseau de transducteurs selon la revendication 10, dans lequel les première et deuxième couches de gel conducteur (106a, b) sont constituées de gels conducteurs différents.

12. Réseau de transducteurs selon la revendication 10, dans lequel les première et deuxième couches de gel conducteur (106a, b) sont faites du même gel conducteur.

13. Réseau de transducteurs selon la revendication 1, dans lequel les éléments de gel conducteur (114) sont des disques dont le diamètre est un peu plus grand que celui des éléments d'électrode (110 ; 110a).

14. Réseau de transducteurs selon la revendication 1, dans lequel les éléments d'électrode (110 ; 110a) sont isolés électriquement.

15. Réseau de transducteurs selon la revendication 1, dans lequel chacun des éléments d'électrode (110 ; 110a) est recouvert d'un matériau diélectrique choisi parmi un matériau céramique, un polymère non flexible ou un film polymère flexible.
